# EUROPEAN PATENT SPECIFICATION

(11) **EP 2 247 585 B1**
(45) Date of publication and mention of the grant of the patent: **30.04.2014**
(21) Application number: 09717772.9
(22) Date of filing: 05.03.2009
(51) Int. Cl.: C07D 403/06, A61K 31/404, A61P 35/00

(54) **CRYSTAL FORMS OF N-[2-(DIETHYLAMINO)ETHYL]-5-[(5-FLUORO-1,2-DIHYDRO-2-OXO-3H-INDOL-3-YLIDENE)METHYL]-2,4-DIMETHYL-1H-PYRROLE-3-CARBOXAMIDE AND METHODS FOR THEIR PREPARATION**
KRISTALLINE FORMEN VON N-[2-(DIETHYLAMINO-)ETHYL]-5-[(5-FLUOR-1,2-DIHYDRO-2-OXO-3H-INDOL-3-YLIDEN-)METHYL]-2,4-DIMETHYL-1H-PYRROL-3-CARBOXAMID UND VERFAHREN ZU IHRER HERSTELLUNG
FORMES CRISTALLINES DU N-[2-(DIÉTHYLAMINO)ÉTHYL]-5-[(5-FLUORO-1,2-DIHYDRO-2-OXO-3H-INDOL-3-YLIDÈNE)MÉTHYL]-2,4-DIMÉTHYL-1H-PYRROL-3-CARBOXAMIDE ET PROCÉDÉS POUR LEUR PRÉPARATION

(30) Priority: 06.03.2008 EP 08004143
(43) Date of publication of application: 10.11.2010
(60) Divisional application: 13158832.9
(73) Proprietor: ratiopharm GmbH, 89079 Ulm (DE)
(72) Inventor: MOHAMAD, Nesrin, 45139 Essen (DE); BOESE, Roland, 45326 Essen (DE); LATZ, Rüdiger, 46047 Oberhausen (DE); STRIEGEL, Hans-Günter, 89143 Blaubeuren (DE)
(74) Representative: Eder, Michael
(86) International application number: PCT/EP2009/001568
(87) International publication number: WO 2009/109388

(56) References cited:
- WO-A-01/60814
- WO-A-03/070725
- WANG ET AL: "Synthesis of [<18>F]SU11248, a new potential PET tracer for imaging cancer tyrosine kinase" BIOORGANIC & MEDICINAL CHEMISTRY LETTERS, OXFORD, GB, vol. 15, no. 19, 1 October 2005 (2005-10-01), pages 4380-4384, XP005038914 ISSN: 0960-894X
- MANLEY JERAD M ET AL: "Early amidation approach to 3-[(4-amido)pyrrol-2-yl]-2-indolinones." THE JOURNAL OF ORGANIC CHEMISTRY 8 AUG 2003, vol. 68, no. 16, 8 August 2003 (2003-08-08), pages 6447-6450, XP007905198 ISSN: 0022-3263
- 'Römp Chemie Lexikon', * page 1977 *
- 'Scientific discussion', [Online] page 3/47 Retrieved from the Internet: <URL:EMEA website - Sutent approval>

## Description

### Field of the invention

The present invention relates to novel crystal forms of Sunitinib, having the chemical name N-[2-(diethylamino)ethyl]-5-[(5-fluoro-1,2-dihydro-2-oxo-3H-indol-3-ylidene)methyl]-2,4-dimethyl-1H-pyrrole-3-carboxamide, and methods for their preparation.

### Prior art and background of the invention

Sunitinib, sold under the brand name Sutent® by Pfizer Pharma, is a receptor tyrosine kinase inhibitor which is used to treat disorders like renal cell carcinoma (RCC) and gastrointestinal stromal tumor (GIST). Its activity relies on the inhibition of cellular signaling by targeting multiple receptor tyrosine kinases including platelet-derived growth factor receptors and vascular endothelial growth factor receptors. Since both kinds of receptors are involved in tumor angiogenesis and tumor cell proliferation, the simultaneous inhibition of these targets results in both reduced tumor vascularization and cancer cell death. These effects are responsible for the finally observed shrinkage of the renal cell carcinoma and gastrointestinal stromal tumor, respectively.

Sunitinib and its pharmaceutical effects on disorders like cancer are described in EP 1255752. Further medical uses of Sunitinib and its salts are inter alia known from EP 1255536 and WO 03/035009.

EP 1255752 discloses Sunitinib and two processes for its preparation. According to these Sunitinib is obtained as a yellow green solid and as an orange solid, respectively. However, when repeating these processes, the yellow green solid cannot be obtained. According to the second process Sunitinib can be obtained.

### Disclosure of the invention

The present invention relates to novel polymorphic forms of N-[2-(diethylamino)ethyl]-5-[(5-fluoro-1,2-dihydro-2-oxo-3H-indol-3-ylidene)methyl]-2,4-dimethyl-1 H-pyrrole-3-carboxamide (Sunitinib) and processes for their preparation. Sunitinib has the following chemical structure:

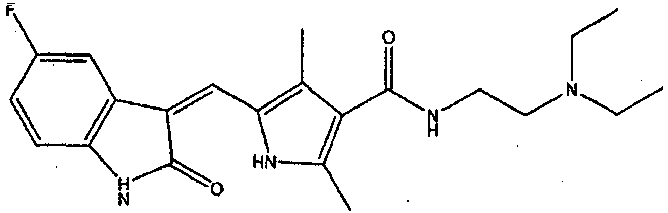

Sunitinib can be obtained in a yellow to greenish yellow powder exhibiting a very low hygroscopicity. In the following, this crystal form of Sunitinib will be designated as "polymorphic form I" or "polymorph I". This powder is fine particulate, hard to collect by filtration, highly electrostatic and tends to strongly stick to all kinds of surfaces.

Furthermore, the present invention describes a polymorphic form II of Sunitinib which does not exhibit the disadvantages of polymorphic form I but is slightly more hygroscopic.

Thirdly, the present invention provides a polymorphic form III of Sunitinib which is not hygroscopic and does not exhibit the disadvantages of polymorphic form I, thereby combining the advantages of polymorphs I and II.

### Abbreviations

- Å: Angström
- DSC: differential scanning calorimetry
- DMSG: dynamic moisture sorption gravimetry
- IR: infrared
- RH: relative humidity
- RT: room temperature
- SCXRD: singe crystal X-ray diffraction
- XRPD: X-ray powder diffraction

### Brief description of the figures

Figure 1: XRPD pattern of Sunitinib in the polymorphic form I
Figure 2: XRPD pattern of Sunitinib in the polymorphic form II
Figure 3: XRPD pattern of Sunitinib in the polymorphic form III
Figure 4: DSC diagram of Sunitinib in the polymorphic form I
Figure 5: DSC diagram of Sunitinib in the polymorphic form II
Figure 6: DSC diagram of Sunitinib in the polymorphic form III
Figure 7: photographs of Sunitinib in the polymorphic forms I, II and III
Figure 8: Raman spectra of Sunitinib in the polymorphic forms I, II and III
Figure 9: IR spectra of Sunitinib in the polymorphic forms I, II and III

### Detailed description of the invention

### Polymorph I of Sunitinib

Described is a yellow to greenish yellow solid , that will in the following be referred to as polymorph I of Sunitinib. It is characterized by an XRPD pattern having a characteristic peak at 4.5 ± 0.2 degrees 2-theta, in particular with peaks at 4.5 ± 0.2; 9.1 ± 0.2; 16.8 ± 0.2 and 26.0 ± 0.2 degrees 2-theta.

Furthermore, polymorph I of Sunitinib can be characterized by an XRPD pattern with peaks at 4.5 ± 0.2; 9.1 ± 0.2; 15.2 ± 0.2; 16.8 ± 0.2; 18.3 ± 0.2; 20.4 ± 0.2; 21.8 ± 0.2; 22.9 ± 0.2 and 26.0 ± 0.2 degrees 2-theta.

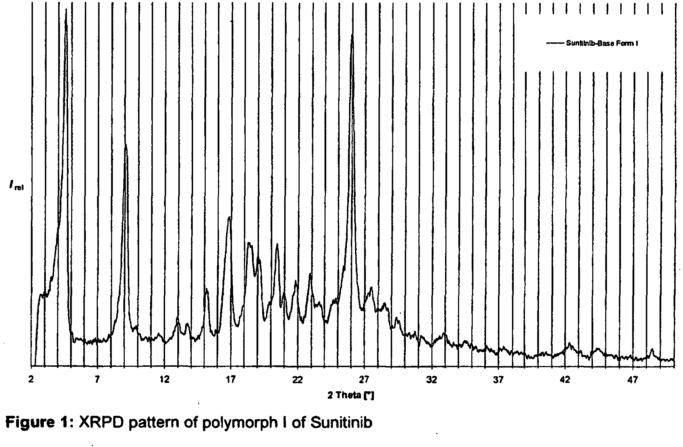

The XRPD pattern of polymorph I of Sunitinib is shown in figure 1.

Polymorph I of Sunitinib shows a Raman spectrum exhibiting characteristic peaks at 2927 ± 2 cm⁻¹, 1679 ± 2 cm⁻¹, 1585 ± 2 cm⁻¹, 1437 ± 2 cm⁻¹, 1334 ± 2 cm⁻¹, 1278 ± 2 cm⁻¹ and 670 ± 2 cm⁻¹.

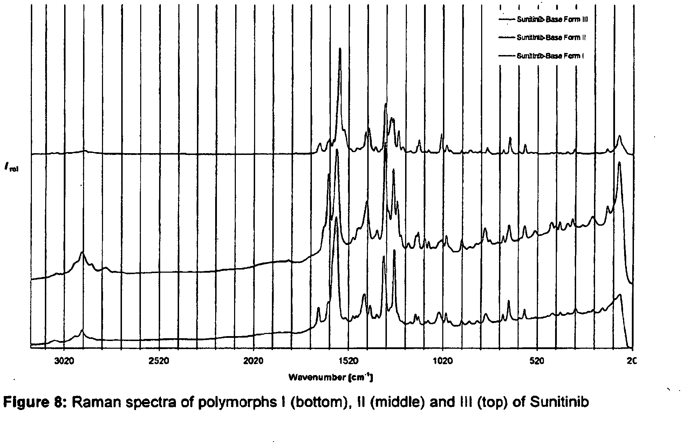

The Raman spectrum of polymorph I of Sunitinib is shown in figure 8.

Polymorph I of Sunitinib shows an IR spectrum exhibiting characteristic peaks at 2969 ± 2 cm⁻¹, 1479 ± 2 cm⁻¹, 1330 ± 2 cm⁻¹, 1189 ± 2 cm⁻¹, 797 ± 2 cm⁻¹, 667 ± 2 cm⁻¹ and 609 ± 2 cm⁻¹.

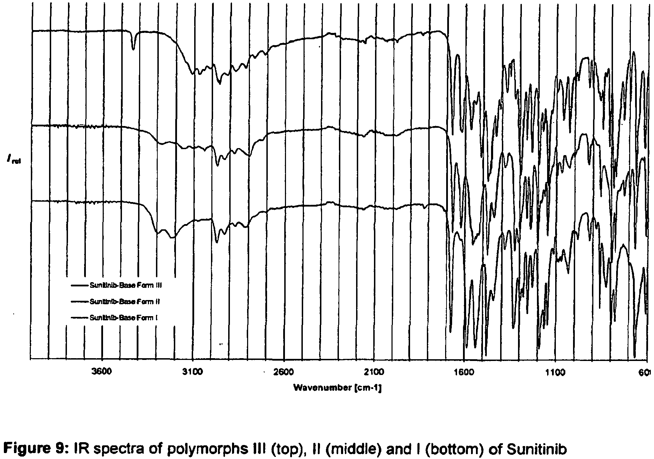

The IR spectrum of polymorph I of Sunitinib is shown in figure 9.

Polymorph I of Sunitinib exhibits a low hygroscopicity. DMSG revealed a moisture sorption of maximum about 1 % referred to the weight of the sample.

Surprisingly, by crystallization and/or recrystallization of Sunitinib from different solvents and mixtures of two or more solvents further new polymorphic forms were found. While polymorph I of Sunitinib exhibits low hygroscopicity it has unfavourable properties such as an extremely fine particularity and bad filterability, stickiness to all sorts of surfaces and electrostatic properties hampering the industrial workability including the development of galenical formulations. In the handling of the further new polymorphs of Sunitinib the above mentioned problems in filterability, electrostatic properties and stickiness were not encountered.

### Polymorph II of Sunitinib

Also described is a polymorph II of Sunitinib. This crystal form has the following characteristics:

| | |
|---|---|
| • crystal system | triclinic |
| • space group | P-1 |
| • cell metrics | a = 13.5 ± 0.2 Å |
| | b = 14.4 ± 0.2 Å |
| | c = 24.3 ± 0.2 Å |
| | α = 79 ± 1° |
| | β = 81 ± 1° |
| | γ = 89 ± 1° |
| • cell volume V | 4598.85 Å³ |
| • molecules per unit cell | 8 |

Polymorph II of Sunitinib is characterized by an XRPD pattern having a characteristic peak at 3.8 ± 0.2 degrees of 2-theta, in particular with peaks at 3.8 ± 0.2; 9.0 ± 0.2; 14.0 ± 0.2; 18.1 ± 0.2 and 20.5 ± 0.2 degrees 2-theta.

Furthermore, polymorph II of Sunitinib can be characterized by an XRPD pattern with peaks at 3.8 ± 0.2; 9.0 ± 0.2; 14.0 ± 0.2; 18.1 ± 0.2; 20.5 ± 0.2; 26.6 ± 0.5 and 27.5 ± 0.6 degrees 2-theta.

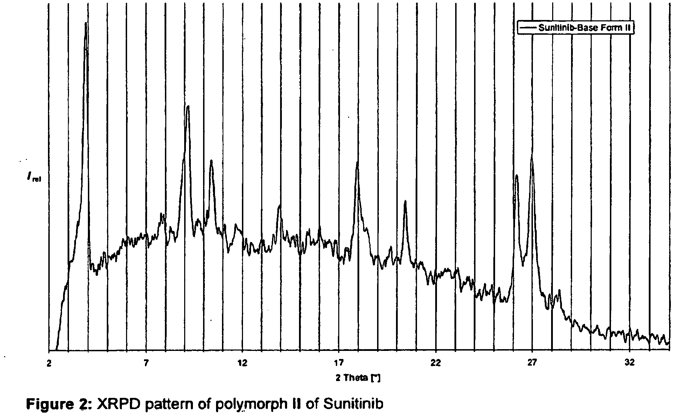

The XRPD of polymorph II of Sunitinib is shown in figure 2.

Polymorph II of Sunitinib shows a Raman spectrum exhibiting characteristic peaks at 2929 ± 2 cm⁻¹, 1627 ± 2 cm⁻¹, 1583 ± 2 cm⁻¹, 1425 ± 2 cm⁻¹, 1328 ± 2 cm⁻¹, 1285 ± 2 cm⁻¹, 1264 ± 2 cm⁻¹ and 669 ± 2 cm⁻¹.

The Raman spectrum of polymorph II of Sunitinib is shown in figure 8.

Polymorph II of Sunitinib shows an IR spectrum exhibiting characteristic peaks at 1667 ± 2 cm⁻¹, 1476 ± 2 cm⁻¹, 1325 ± 2 cm⁻¹, 1147 ± 2 cm⁻¹, 794 ± 2 cm⁻¹, 668 ± 2 cm⁻¹ and 608 ± 2 cm⁻¹.

The IR spectrum of polymorph II of Sunitinib is shown in figure 9.

Sunitinib in the polymorphic form II exhibits some hygroscopicity. DMSG revealed a moisture sorption of more than 6 % referred to the weight of the sample.

Also described is a process for the preparation of polymorph II of Sunitinib, comprising the steps of:
- dissolving Sunitinib present in any polymorphic or amorphous form or mixtures thereof, preferably in its polymorphic form I in a suitable inert solvent, preferably in water or an organic solvent or a mixture of two or more suitable inert solvents, preferably at a temperature between 25 °C and the reflux temperature of the solvent or the solvent mixture,
- optionally adding another solvent, preferably another organic solvent,
- concentrating the solution, preferably under reduced pressure, more preferably at a temperature between 25 °C and the reflux temperature of the solvent or the solvent mixture, most preferably under reduced pressure at 25 to 60 °C,
- collecting the resulting crystalline precipitate, preferably by filtration.

The crystallization is carried out in a suitable Inert solvent or in a mixture of two or more suitable inert solvents. Examples of such suitable inert solvents are water, aliphatic and aromatic hydrocarbons (preferably hexane, benzene, toluene or xylene), aliphatic alcohols (preferably methanol, ethanol, propanol, iso-propanol), ethers (preferably diethyl ether, diisopropyl ether or dimethoxyethane), cyclic ethers (preferably tetrahydrofuran or dioxane), ketones (preferably acetone, methylisobutylketone or methylethylketone), esters (preferably ethylacetate), chlorinated hydrocharbons (preferably dichloromethane or chloroform) or nitrogen containing organic solvents (preferably N-methyl pyrollidone, dimethylformamide or acetonitrile). Acetone, methylethylketone and toluene are especially preferred.

A further alternative process for the preparation of polymorph II of Sunitinib is described, comprising the steps of:
- dissolving a salt of Sunitinib, preferably a salt of Sunitinib (for example as disclosed in EP 1255752 B1), more preferably the malate salt, in a suitable inert solvent, preferably in water, or a mixture of two or more suitable inert solvents optionally heating the solvent to a temperature between 25 °C and the reflux temperature of the solvent or the solvent mixture,
- adding a base, preferably NaOH,
- collecting the resulting crystalline precipitate, preferably by filtration.

As solvents the suitable inert solvents as described above can be used.

The above described reaction is carried out in the presence of a base, preferably a Bronsted base. Examples of suitable Bronsted bases are metal hydroxides, metal carbonates or amines, preferably alkali metal hydroxides, alkali metal carbonates, alkali metal bicarbonates, ammonia, or organic amines, such as, for example, sodium hydroxide, potassium hydroxide, sodium carbonate, potassium carbonate, sodium bicarbonate, ammonia, diethylamine, triethylamine, diisopropylamine or pyridine. Sodium hydroxide is preferred. The base is used in an amount sufficient to obtain the free base of Sunitinib from its salt.

### Polymorph III of Sunitinib

One embodiment of the present invention relates to polymorph III of Sunitinib. This crystal form has the following characteristics:

| | |
|---|---|
| • crystal system | monoclinic |
| • space group | P 2₁/n |
| • cell metrics | a = 4.97560(10) Å |
| | b = 28.1365(6) Å |
| | c = 14.5880(3) Å |
| | β = 93.5130(10) ° |
| • cell volume V | 2038.42(7) Å³ |
| • molecules per unit cell | 4 |

Polymorph III of Sunitinib is characterized by an XRPD pattern having a characteristic peak at 6.3 ± 0.2 degrees 2-theta, in particular with peaks at 6.3 ± 0.2; 22.2 ± 0.2 and 26.4 ± 0.2 degrees 2-theta.

Furthermore, polymorph III of Sunitinib can be characterized by an XRPD pattern with peaks at 6.3±0.2; 14.0±0.2; 15.4±0.2, 18.9±0.2, 19.3±0.2; 22.2±0.2; 24.2±0.2 and 26.4 ± 0.2 degrees 2-theta.

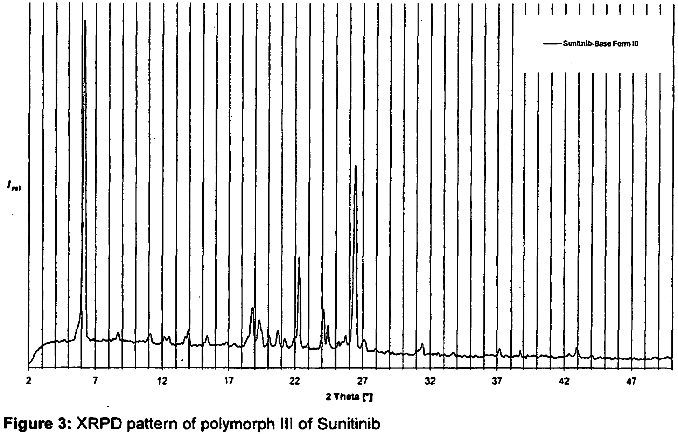

The XRPD pattern of polymorph III of Sunitinib is shown in figure 3.

Polymorph III of Sunitinib shows a Raman spectrum exhibiting characteristic peaks at 1674 ± 2 cm⁻¹, 1569 ± 2 cm⁻¹, 1414 ± 2 cm⁻¹, 1327 ± 2 cm⁻¹, 1297 ± 2 cm⁻¹, 1259 ± 2 cm⁻¹, 1030 ± 2 cm⁻¹ and 666 ± 2 cm⁻¹.

The Raman spectrum of polymorph III of Sunitinib is shown in figure 8.

Polymorph III of Sunitinib shows an IR spectrum exhibiting characteristic peaks at 3435 ± 2 cm⁻¹, 1670 ± 2 cm⁻¹, 1473 ± 2 cm⁻¹, 1294 ± 2 cm⁻¹, 1194 ± 2 cm⁻¹, 1146 ± 2 cm⁻¹, 786 ± 2 cm⁻¹, 663 ± 2 cm⁻¹, 617 ± 2 cm⁻¹.

The IR spectrum of polymorph III of Sunitinib is shown in figure 9.

Polymorph III exhibits a very low hygroscopicity. Under DMSG, the sample does not gain weight, even at a relative humidity of 95 %.

Polymorph III is preferably further characterized by the absence of any solvent molecules within the crystal.

The present invention also relates to a process for the preparation of polymorph III of Sunitinib, comprising the steps of:
- preparing a clear saturated solution of Sunitinib in a suitable inert solvent,
- cooling the solution and allowing Sunitinib to crystallize,
- removing the resulting precipitate,
- keeping the filtrate at room temperature until Sunitinib crystallizes in the form of dark orange needles
   or alternatively:
- preparing a clear saturated solution of Sunitinib in a suitable inert solvent,
- cooling the solution and seeding it with crystals of polymorph III of Sunitinib,
- allowing polymorph III to crystallize from the seeded solution at room temperature in the form of dark orange needles.

Preferably the process comprises the steps of:
- preparing a suspension of Sunitinib by suspending Sunitinib present in any polymorphic or amorphous form or mixtures thereof, preferably in its polymorphic form I or II, in a suitable inert solvent, preferably in an organic solvent or a mixture of two or more organic solvents,
- heating the suspension, preferably to the reflux temperature of the solvent or the solvent mixture, optionally by adding an additional amount of solvent, until a clear and saturated solution is obtained,
- cooling the solution and allowing Sunitinib to precipitate,
- removing the resulting precipitate, preferably by filtration,
- keeping the filtrate at room temperature until Sunitinib crystallizes in the form of dark orange needles,
   or alternatively:
- preparing a suspension of Sunitinib by suspending Sunitinib present in any polymorphic or amorphous form or mixtures thereof, preferably in its polymorphic form I or II in a suitable inert solvent, preferably in an organic solvent or a mixture of two or more organic solvents,
- heating the saturated suspension, preferably to the reflux temperature of the solvent or the solvent mixture, optionally by adding an additional amount of solvent, until a clear and saturated solution is obtained,
- cooling the solution and seeding it with crystals of polymorph III of Sunitinib,
- allowing polymorph III of Sunitinib to crystallize from the seeded solution at room temperature in the form of dark orange needles.

The above described crystallizations are carried out in suitable inert solvents or mixtures of two or more suitable inert solvents. Examples of such suitable inert solvents are water, aliphatic and aromatic hydrocarbons (preferably hexane, benzene, toluene or xylene), aliphatic alcohols (preferably methanol, ethanol, propanol, iso-propanol), ethers (preferably diethyl ether, diisopropyl ether or dimethoxyethane), cyclic ethers (preferably tetrahydrofuran or dioxane), ketones (preferably acetone, methylisobutylketone or methylethylketone), esters (preferably ethylacetate), chlorinated hydrocharbons (preferably dichloromethane or chloroform) or nitrogen containing organic solvents (preferably N-methyl pyrollidone, dimethylformamide or acetonitrile). Ketones, such as acetone and methylethylketone, as well as toluene are especially preferred.

In a preferred embodiment of the process for the preparation of polymorph III of Sunitinib of the present invention the seeding of the solution with crystals of polymorph III of Sunitinib is carried out at a temperature of the solution of about 40°C to about 60°C, preferably of about 50°C. In this embodiment the preferred inert solvents are ketones, in particular methylethylketone.

### Comparative analysis of the 3 polymorphs

Figures 1, 2 and 3 show that the polymorphs I, II and III of Sunitinib can clearly be identified by their XRPD patterns.

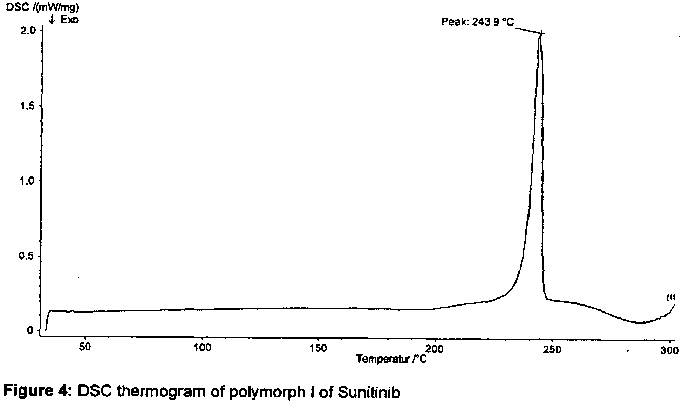

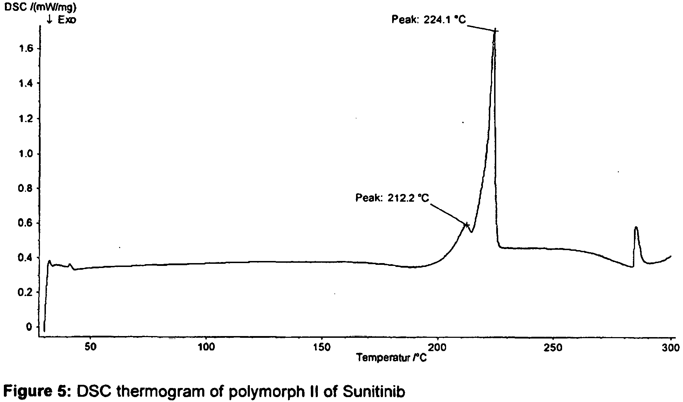

The DSC thermogram in figure 4 shows that polymorph I has a melting point of approximately 244 °C that differs significantly from that of polymorph II (224 °C) shown in figure 5.

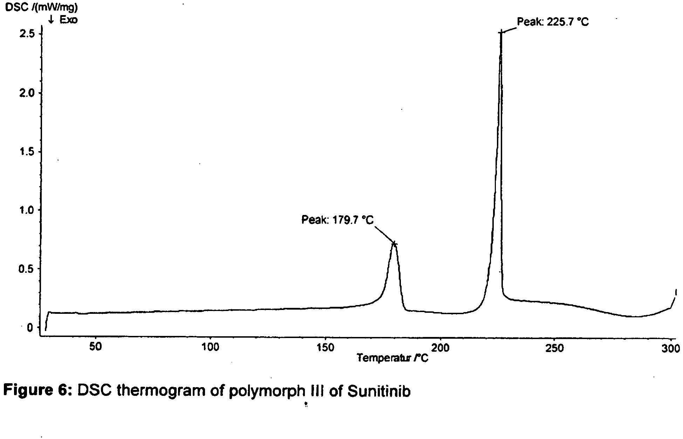

The DSC thermogram in figure 6 reveals an endothermic signal at approximately 180 °C indicating a change in the solid state form of polymorph III of Sunitinib. The resulting form shows a melting point of approximately 226 °C which is similiar to the one seen for polymorph II. This provides evidence for the fact that polymorph III can be transformed to polymorph II via an endothermic process. The above used term "approximately" is owed to the fact that there is an uncertainty in the measurement of DSC thermograms in the range of ± 4 °C.

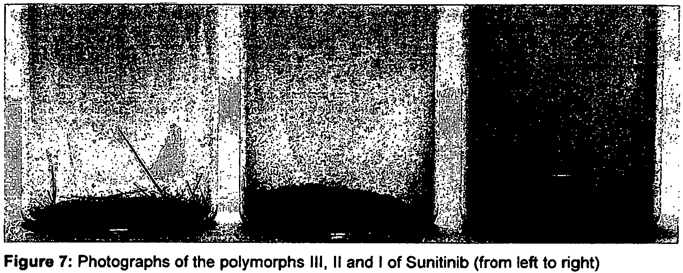

Photographs of the 3 polymorphic forms of Sunitinib are shown in figure 7. Polymorph III of Sunitinib exhibits needle shaped dark orange crystals, whereas polymorphs I and **II** are obtained as powders. Polymorph II is light orange, while polymorph I is yellow to greenish yellow. The picture also illustrates the electrostatic and sticking properties of polymorph I (the substance covers the inner surface of the glass container) making it difficult to handle, e.g. to transfer, to weigh, to process or to mix with excipients.

DMSG data show that polymorph I is only slightly hygroscopic, which is a clear advantage over polymorph II, which is clearly hygroscopic. Polymorph III is not hygroscopic at all.

Polymorph I is only very slightly hygroscopic and polymorph II does not show the disadvantageous electrostatic properties of polymorph I. Thus, either of them possesses certain advantageous properties. For example, if used in pharmaceutical formulations for parenteral application, hygroscopicity is not a problem since these are prepared as liquids. Thus, polymorph II is advantageous over polymorph I for the preparation of parenteral dosage forms. On the other hand, electrostatic properties can be overcome by adding suitable excipients e.g. during a wet granulation process or the preparation of topical medicaments. Therefore polymorph I might be advantageous over polymorph II for the preparation of solid or semisolid dosage forms.

Polymorph III combines the advantages of the polymorphs I and II and represents an embodiment of the present invention.

The invention is further illustrated by the following examples which are not constructed as being limiting.

### Examples

XRPD was performed on a STOE Transmission Diffractometer STADE P (2003-10) using the Kα1 band of a copper radiation source.

SCXRD was performed under the following measurement conditions:

| | |
|---|---|
| Diffractometer control software: | Bruker AXS APEX 2 |
| Diffractometer measurement device | Siemens Smart three axis goniometer with APEX area detector system |
| Diffractometer measurement method | Data collection strategy APEX 2/COSMO chi±5° |
| Computing data reduction | Bruker AXS APEX 2 |
| Empirical absorption correction | Bruker AXS APEX 2 |
| Computing structure solution | Bruker AXS SHELTXTL |
| Computing structure refinement | Bruker AXS |

Raman spectroscopy was performed on a Bruker IFS66 Raman Spectrometer (FRA 106).

DSC was performed on a DSC 204 Phoenix calorimeter (NETZSCH Gerätelabor GmbH) using closed aluminium crucibles under N₂ atmosphere (0.45 bar) with a cooling/heating rate of 5 °C/minute.

IR spectroscopy was performed on a Varian 3100 FT-IR-Spectrometer (Excalibur Series) using the ATR sampling technique.

DMSG was performed using an SPS11-100n instrument (Projekt Messtechnik) at a constant temperature of 25 °C and a change in RH of 5 % per hour including isohumid conditions for 5 hours at 0 % RH and for 2 hours at 50 % RH.

### Comparative Example 1: preparation of polymorph II of Sunitinib

Sunitinib was obtained according to example 35 (alternative synthesis) of EP 1255752 B1. 0.25 g of Sunitinib were mixed with 50 ml acetone and heated to reflux. Then, 5 ml toluene were added. The solvent was removed at 45 °C under reduced pressure of 250 to 300 mmHg until precipitation of the crystalline solid occurred. The product was collected by filtration, washed with 2 ml of cold toluene and dried for 24 hours at room temperature.

### Example 2: preparation of polymorph III of Sunitinib

Sunitinib was obtained according to example 35 (alternative synthesis) of EP 1255752 B1. A suspension of Sunitinib was prepared in a mixture of acetone and toluene (1:1 v/v) at room temperature (RT). The suspension was heated to reflux for 1 hour, leading to a complete dissolution of Sunitinib. The solution was allowed to cool below boiling temperature and was then further cooled with a mixture of ice and sodium chloride as external cooling for 24 hours. The ice bath was not exchanged and the mixture was allowed to warm to RT. Further crystallization at RT for 48 hours resulted in a yellow precipitate, which was removed by filtration. The dark orange filtrate was kept at RT until dark orange needles were formed. These were isolated and dried for 24 hours at RT.

### Example 3: preparation of polymorph III of Sunitinib

Sunitinib form II was refluxed in acetone to get a saturated solution. Undissolved solids were filtered off while hot and the clear solution was seeded with form III. The solvent was partially evaporated under vacuum using a water bath tempered at 30-40°C and the residual solution was cooled to room temperature. The precipitate was filtered off, washed with acetone and dried under vacuum yielding Sunitinib free base as crystalline form III.

### Example 4: preparation of polymorph III of Sunitinib

Sunitinib base form II was refluxed (80°C) in methylethylketone to get a saturated solution (approximately 6.5 g of base and 20 ml of methylethylketone). Undissolved solids were filtered off while hot and the clear solution was seeded with form III (approximately 50°C). The solvent was partially evaporated under vacuum using a water bath tempered at 30-40°C and the residual solution was cooled to room temperature. The precipitate was filtered off, washed with methylethylketone and dried under vacuum yielding Sunitinib free base as crystalline form III.

## Claims

1. Sunitinib polymorph III **characterized by** an XRPD pattern having characteristic peaks at 6.3 ± 0.2; 22.2 ± 0.2 and 26.4 ± 0.2 degrees 2-theta.

2. Sunitinib polymorph III according to claim 1, **characterized by** having the following characteristics:
• crystal system monoclinic
• space group P 2₁/n
• cell metrics a = 4.97560(10) Å; b = 28.1365(6) Å
c = 14.5880(3) Å; β = 93.5130(10)°
• cell volume V 2038.42(7) Å³
• molecules per unit cell 4.

3. Sunitinib polymorph III according to claim 1 or 2, **characterized by** having an XRPD pattern having a characteristic peak at 6.3 ± 0.2; 14.0 ± 0.2; 15.4 ± 0.2, 18.9 ± 0.2, 19.3 ± 0.2; 22.2 ± 0.2; 24.2 ± 0.2 and 26.4 ± 0.2 degrees 2-theta.

4. Sunitinib polymorph III according to any one of the preceding claims, **characterized by** having an XRPD pattern depicted in figure 3.

5. Sunitinib polymorph III according to any one of the preceding claims, **characterized by** having a Raman spectrum exhibiting characteristic peaks at 1674 ± 2 cm⁻¹, 1569 ± 2 cm⁻¹ , 1414 ± 2 cm⁻¹, 1327 ±2 cm⁻¹, 1297 ± 2 cm⁻¹, 1259 ± 2 cm⁻¹, 1030 ± 2 cm⁻¹ and 666 ± 2 cm⁻¹.

6. Sunitinib polymorph III according to any one of the preceding claims, **characterized by** having a Raman spectrum as depicted in figure 8.

7. Sunitinib polymorph III according to any one of the preceding claims, **characterized by** having an IR spectrum exhibiting characteristic peaks at 3435 ± 2 cm⁻¹, 1670 ± 2 cm⁻¹, 1473 ± 2 cm⁻¹, 1294 ± 2 cm⁻¹, 1194 ± 2 cm⁻¹, 1146 ± 2 cm⁻¹, 786 ± 2 cm⁻¹, 663 ± 2 cm⁻¹ 617 ± 2 cm⁻¹.

8. Sunitinib polymorph III according to any one of the preceding claims, **characterized by** having an IR spectrum as depicted in figure 9.

9. Process for the preparation of polymorph III of Sunitinib as defined in any one of the preceding claims, comprising the steps of:
• preparing a clear saturated solution of Sunitinib in a suitable inert solvent,
• cooling the solution and allowing Sunitinib to crystallize,
• removing the resulting precipitate,
• keeping the filtrate at room temperature until Sunitinib crystallizes in the form of dark orange needles or alternatively:
• preparing a clear saturated solution of Sunitinib in a suitable inert solvent,
• cooling the solution and seeding it with crystals of polymorph III of Sunitinib,
• allowing polymorph III to crystallize from the seeded solution at room temperature in the form of dark orange needles.

10. Process according to claim 9, wherein the inert solvent is a ketone, preferably methylethylketone.

11. Process according to claim 9 or 10, wherein the solution is cooled to a temperature in the range of about 40°C to about 60°C before seeding it with crystals of polymorph III of Sunitinib.

12. Use of polymorph III of Sunitinib according to any one of claims 1 to 8 for the preparation of a pharmaceutical preparation.

13. Pharmaceutical preparation comprising a polymorph III of Sunitinib according any one of claims 1 to 8.

## Patentansprüche

1. Sunitinib-Polymorph III, **gekennzeichnet durch** ein XRPD-Muster, das charakteristische Spitzenwerte (Peaks) bei 6,3 ± 0,2; 22,2 ± 0,2 und 26,4 ± 0,2 Grad 2-Theta aufweist.

2. Sunitinib-Polymorph III nach Anspruch 1, **dadurch gekennzeichnet, dass** es die folgenden Eigenschaften aufweist:
| | |
|---|---|
| • Kristallsystem | monoklin |
| • Raumgruppe | P 2₁/n |
| • Zellmetrik | a = 4,97560(10) Å; b = 28,1365(6) Å |
| | c = 14,5880(3) Å; β = 93,5130(10)° |
| • Zellvolumen V | 2038,42(7) Å³ |
| • Moleküle pro Einheitszelle | 4. |

3. Sunitinib-Polymorph III nach Anspruch 1 oder 2, **gekennzeichnet dadurch, dass** es ein XRPD-Muster mit einem charakteristischen Spitzenwert (Peak) bei 6,3 ± 0,2; 14,0 ± 0,2; 15,4 ± 0,2, 18,9 ± 0,2, 19,3 ± 0,2; 22,2 ± 0,2; 24,2 ± 0,2 und 26,4 ± 0,2 Grad 2-Theta aufweist.

4. Sunitinib-Polymorph III nach einem der vorhergehenden Ansprüche, **dadurch gekennzeichnet, dass** es ein in Figur 3 dargestelltes XRPD-Muster aufweist.

5. Sunitinib-Polymorph III nach einem der vorhergehenden Ansprüche, **dadurch gekennzeichnet, dass** es ein Raman-Spektrum aufweist, das charakteristische Spitzenwerte (Peaks) bei 1674 ± 2 cm⁻¹, 1569 ± 2 cm⁻¹, 1414 ± 2 cm⁻¹, 1327 ± 2 cm⁻¹, 1297 ± 2 cm⁻¹, 1259 ± 2 cm⁻¹, 1030 ± 2 cm⁻¹ und 666 ± 2 cm⁻¹ aufweist.

6. Sunitinib-Polymorph III nach einem der vorhergehenden Ansprüche, **dadurch gekennzeichnet, dass** es ein wie in Figur 8 dargestelltes Raman-Spektrum aufweist.

7. Sunitinib-Polymorph III nach einem der vorhergehenden Ansprüche, **dadurch gekennzeichnet, dass** es ein IR-Spektrum aufweist, das charakteristische Spitzenwerte (Peaks) bei 3435 ± 2 cm⁻¹, 1670 ± 2 cm⁻¹, 1473 ± 2 cm⁻¹, 1294 ± 2 cm⁻¹, 1194 ± 2 cm⁻¹, 1146 ± 2 cm⁻¹, 786 ± 2 cm⁻¹, 663 ± 2 cm⁻¹, 617 ± 2 cm⁻¹ aufweist.

8. Sunitinib-Polymorph III nach einem der vorhergehenden Ansprüche, **dadurch gekennzeichnet, dass** es ein IR-Spektrum wie in Figur 9 dargestellt aufweist.

9. Prozess für die Herstellung von Polymorph III von Sunitinib, wie in einem der vorhergehenden Ansprüche definiert, umfassend die folgenden Schritte:
• Herstellen einer klaren gesättigten Lösung von Sunitinib in einem geeigneten inerten Lösungsmittel,
• Kühlen der Lösung und Kristallisierenlassen von Sunitinib,
• Entfernen des resultierenden Niederschlags,
• Halten des Filtrats bei Raumtemperatur, bis Sunitinib in Form von dunkelorangen Nadeln kristallisiert, oder alternativ:
• Herstellen einer klaren gesättigten Lösung von Sunitinib in einem geeigneten inerten Lösungsmittel,
• Kühlen der Lösung und Versetzen der Lösung mit Saatkristallen von Polymorph III von Sunitinib,
• Kristallisierenlassen von Polymorph III aus der mit Saatkristallen versetzten Lösung bei Raumtemperatur in der Form von dunkelorangen Nadeln.

10. Prozess nach Anspruch 9, wobei es sich bei dem inerten Lösungsmittel um ein Keton handelt, vorzugsweise um Methylethylketon.

11. Prozess nach Anspruch 9 oder 10, wobei die Lösung auf eine Temperatur im Bereich von etwa 40 °C bis etwa 60 °C gekühlt wird, bevor sie mit Saatkristallen von Polymorph III von Sunitinib versetzt wird.

12. Verwendung von Polymorph III von Sunitinib nach einem der Ansprüche 1 bis 8 für die Herstellung einer pharmazeutischen Zubereitung.

13. Pharmazeutische Zubereitung, umfassend ein Polymorph III von Sunitinib nach einem der Ansprüche 1 bis 8.

## Revendications

1. Polymorphe III de Sunitinib **caractérisé par** XRPD ayant des pics caractéristiques à 6,3 ± 0,2 ; 22,2 ± 0,2 et 26,4 ± 0,2 degrés de 2-thêta.

2. Polymorphe III de Sunitinib selon la revendication 1, ayant les caractéristiques suivantes :
| | |
|---|---|
| • système cristallin | monoclinique |
| • groupe spatial | P 2₁/n |
| • Mesures cellulaires | a = 4,97560(10) Å ; b = 28,1365(6) Å |
| | c = 14,5880(3) Å ; β = 93,5130(10)° |
| • Volume cellulaire V | 2038,42(7) Å³ |
| • molécules par cellule unitaire | 4. |

3. Polymorphe III de Sunitinib selon la revendication 1 ou la revendication 2, **caractérisé en ce qu'**il a un motif XRPD ayant un pic caractéristique à 6,3 ± 0,2 ; 14,0 ± 0,2 ; 15,4 ± 0,2 ; 18,9 ± 0,2 ; 19,3 ± 0,2 ; 22,2 ± 0,2 ; 24,2 ± 0,2 et 26,4 ± 0,2 degrés de 2-thêta.

4. Polymorphe III de Sunitinib selon l'une quelconque des revendications précédentes, **caractérisé en ce qu'**il a un motif XRPD illustré à la figure 3.

5. Polymorphe III de Sunitinib selon l'une quelconque des revendications précédentes, **caractérisé en ce qu'**il a un spectre Raman exhibant des pics caractéristiques à 1674 ± 2 cm⁻¹, 1569 ± 2 cm⁻¹, 1414 ± 2 cm⁻¹, 1327 ± 2 cm⁻¹, 1297 ± 2 cm⁻¹, 1259 ± 2 cm⁻¹, 1030 ± 2 cm⁻¹ et 666 ± 2 cm⁻¹.

6. Polymorphe III de Sunitinib selon l'une quelconque des revendications précédentes, **caractérisé en ce qu'**il a un spectre Raman tel qu'illustré à la figure 8.

7. Polymorphe III de Sunitinib selon l'une quelconque des revendications précédentes, **caractérisé en ce qu'**il a un spectre à IR exhibant des pics caractéristiques à 3435 ± 2 cm⁻¹, 1670 ± 2 cm⁻¹, 1473 ± 2 cm⁻¹, 1294 ± 2 cm⁻¹, 1194 ± 2 cm⁻¹, 1146 ± 2 cm⁻¹, 786 ± 2 cm⁻¹, 663 ± 2 cm⁻¹, 617 ± 2 cm⁻¹.

8. Polymorphe III de Sunitinib selon l'une quelconque des revendications précédentes, **caractérisé en ce qu'**il a un spectre à IR tel qu'illustré à la figure 9.

9. Processus de préparation du polymorphe III de Sunitinib tel que défini selon l'une quelconque des revendications précédentes, comprenant les étapes consistant à :
• préparer une solution saturée claire de Sunitinib dans un solvant inerte approprié,
• refroidir la solution et permettre au Sunitinib de cristalliser,
• éliminer le précipité résultant,
• maintenir le filtrat à la température ambiante jusqu'à ce que le Sunitinib cristallise sous la forme d'aiguilles orange foncé ou de façon alternative :
• préparer une solution saturée claire de Sunitinib dans un solvant inerte approprié,
• refroidir la solution et l'ensemencée avec des cristaux de polymorphe III de Sunitinib,
• laisser le polymorphe III cristalliser à partir de la solution ensemencée à la température ambiante sous la forme d'aiguilles orange foncé.

10. Processus selon la revendication 9, dans lequel le solvant inerte est une cétone, de préférence une méthyléthylcétone.

11. Processus selon la revendication 9 ou la revendication 10, dans lequel la solution est refroidie à une température dans la gamme d'environ 40° C à environ 60° C avant de l'ensemencer avec des cristaux de polymorphe III de Sunitinib.

12. Utilisation du polymorphe III de Sunitinib selon l'une quelconque des revendications 1 à 8 pour la préparation d'une préparation pharmaceutique.

13. Préparation pharmaceutique comprenant un polymorphe III de Sunitinib selon l'une quelconque des revendications 1 à 8.
